# EUROPEAN PATENT SPECIFICATION

(11) **EP 0 834 288 B1**
(45) Date of publication and mention of the grant of the patent: **27.03.2002**
(21) Application number: 97307827.2
(22) Date of filing: 03.10.1997
(51) Int. Cl.: A61B 18/14

(54) **Apparatus for the thermal treatment of tissue**
Vorrichtung zur thermischen Behandlung von Gewebe
Appareil de traitement thermique des tissus

(30) Priority: 04.10.1996 US 27600 P
(43) Date of publication of application: 08.04.1998
(73) Proprietor: UNITED STATES SURGICAL CORPORATION, Norwalk, Connecticut 06850 (US)
(72) Inventor: Blewett, Jeffrey J., Plantsville, CT 06479 (US); Maurer, Christopher W., Newtown, CT 06470 (US); Stone, Corbett W., San Diego, CA 92131 (US)
(74) Representative: Woodward, John Calvin

(56) References cited:
- EP-A- 0 629 382
- EP-A- 0 667 126
- WO-A-94/17856
- DE-U- 9 419 137
- US-A- 5 472 441

## Description

The present invention relates generally to an apparatus for thermal treatment of tissue and, more particularly, to an auxiliary apparatus to be used with a conventional endoscope to provide the endoscope with thermal treatment capabilities. The apparatus is also capable for use with a cytoscope or urethroscope for hyperthermia treatment for prostatic tissue. An apparatus for the thermal treatment of tissue is known from WO-A-9417856, and comprises a housing dimensioned to be grasped with the hand of a user, and an elongate portion connected to the housing extending distally therefrom, and dimensioned for insertion within a narrow body passage and defining a longitudinal axis, said elongate portion including at least one delivery catheter having proximal and distal end portions, the or each delivery catheter being movable relative to the housing in a generally longitudinal direction between a retracted position and an advanced position, an electromagnetic probe disposed within the or each delivery catheter and movable in a general longitudinal direction therein to extend a probe end portion thereof beyond the distal end portion of the delivery catheter and within the tissue, the electromagnetic probe being adapted to follow a path defined by the distal end portion of the delivery catheter and being connected to a thermal energy source, and a rotatable control member mounted to the housing and operatively connected to the delivery catheter operable to move the delivery catheter between the retracted position and the advanced position, as in the first part of claim 1.

Benign prostrate hyperplasia (BPH) or hyperplasia affects over one out of every two males over the ages of fifty. BPH is the non-cancerous enlargement of the prostrate gland and is characterised generally by a constriction of the uretha by the prostate gland. An array of symptoms are associated with BPH including frequent urination, complications in urinary flow and associated pain.

Generally there are two primary methods for treating BPH, namely, drug therapy and surgical intervention. Drug therapy incorporates the use of one or more drugs such as Proscar™ and Hydrin™ to either reduce the size of the prostate or to relax the urethal muscles thereby facilitating the normal functioning of the urinary system. Known drug therapies, however, are limited in their effectiveness and present many drug side effect concerns.

Surgical methods for treating BPH include transurethal resection of the prostate (TURP), transurethal incision of the prostate (TUIP), visual laser assisted prostatectomy (VLAP), balloon dilation and stenting. TURP is the most common method employed for BPH treatment today and involves the insertion of an electrosurgical cutting instrument through the urethal passage. The cutting elements of the instrument are positioned adjacent the prostate gland, and the instrument is energised such that the cutting elements selectively cauterize and resect tissue from the core of the prostate. The TURP procedure, however, has many side effects including bleeding, retrograde ejaculation, impotence, incontinence, edema and a prolonged recovery period for the patient. An example of an electrosurgical cutting instrument utilized in conjunction with a TURP procedure is disclosed in US Patent No. 5192280.

Transurethral incision of the prostate (TUIP) involves the use of an electrocautery device which is passed through the urethra. The device is employed to make multiple incisions in the prostate, thereby permitting the prostate to be displaced from the urethra wall to create an opening for urine flow. Success with the TUIP procedure is generally limited providing only temporary relief and requiring a subsequent repeat of the procedure in the future.

Visual laser assisted prostatectomy (VLAP) includes insertion of a laser catheter through the urethra and directing laser energy laterally through the catheter sleeve at the urethral wall and the prostatic tissue. The laser energy causes the tissue to coagulate. The coagulated tissue eventually necrosis from lack of blood flow and is naturally removed from the body. Drawbacks of VLAP include increased recovery time, acute pain and irritation, and undesired burning of the urethral wall. Examples of methods and apparatuses utilized in VLAP treatment of BPH are disclosed in US Patent No. 5242438 to Saadatmanesh et al. and US Patent No. 5322507 to Costello.

Balloon dilation procedures for BPH involve expanding and stretching the enlarged prostate with a balloon catheter to relieve pressure off the constricted urethra while stenting incorporates the insertion of tiny wire-mesh coils which expand into a scaffold to hold the urethra open. Balloon dilation and stenting, however, are only temporary procedures typically requiring follow up within a year period. In addition, stenting presents complications of stent migration and consequent irritation.

Transurethral microwave therapy (TUMT) and high intensity focused ultrasound (HIFU) have been developed for the treatment of BPH. In accordance with a TUMT procedure, a foley-type urethral catheter having a microwave emitting antenna at a probe end is inserted into the urethral passage for a period of time sufficient to treat the tissue by microwave radiation. Intraurethral applicators of this type are described in US Patent Nos. 4967765, 5234004 and 5326343. The drawbacks of TUMT include the inability to focus the heat energy in the prostatic area and the inability to achieve high temperatures uniformly within the prostate.

High intensity focused ultrasound (HIFU) includes directing high intensity ultrasound waves at the prostate tissue to create heat in a precise area to coagulate and necrose tissue. A transurethral probe is utilized to create the ultrasound beams for both imaging and ablation of the prostatic tissue. Disadvantages of this procedure include the inability to directly focus the ultrasound energy at the prostatic tissue.

A more recent form of treatment for BPH involves thermally treating prostatic tissue with radio frequency electromagnetic energy. For example, one current technique, known as transurethral needle ablation (TUNA™), involves the transurethral application of a medical instrument having a built-in RF needle electrode system. The TUNA™ instrument is inserted into the urethra and advanced to a position adjacent the prostate. Thereafter, the RF needles are advanced to penetrate the urethral wall and access the prostatic tissue. The RF system is activated whereby a RF current is transmitted through each electrode to pass through the tissue to a grounding pad thereby forming a necrotic legion which is eventually absorbed by the body. Apparatuses and methods for treating BPH via the TUNA™ technique are disclosed for example in US Patent No. 5366490.

The TUNA technique has several disadvantages which detract from its usefulness. In particular, the TUNA instruments are generally complex typically incorporating built in optical systems, aspiration systems etc. As a result, the instruments are relatively expensive to manufacture. Moreover, the TUNA instruments are generally enlarged by virtue of the various systems incorporated within the instrument, thus, increasing patient trauma and discomfort during use.

Accordingly, the present invention seeks to provide an auxiliary apparatus for the RF thermal treatment of prostaic tissue. This apparatus is intended for use in conjunction with a conventional endoscope such as a cytoscope and incorporates an RF system and associated mechanism that is at least partially positionable within the working channel of the scope. The apparatus by use in conjunction with a conventional cytoscope makes use of the existing systems, e.g. optical and illumination, of the scope, which effectively results in a less complex and less expensive RF thermal treatment device. Furthermore, the apparatus may be used in cytoscopes as small as 5mm (or even smaller) in diameter thereby providing a less invasive system for transurethal ablation as compared to the TUNA instruments and technique.

Apparatus for the thermal treatment of tissue according to the present invention as set out in claim 1 is characterised in that the housing is divided into a stationary portion having an externally threaded portion and a movable portion including a groove and a deployment member for the electromagnetic probe, the control member having an internal thread for engagement with the externally threaded portion and being longitudinally fixed to the movable portion of the housing by means of a collar in engagement with the groove, rotation of the control member causing movement of the delivery catheter between the retracted and the advanced position.

In a preferred embodiment, the auxiliary apparatus includes a handle portion and an elongate portion connected to the handle portion and dimensioned to be at least partially inserted within a working channel of an endoscope. The elongate portion includes at least one delivery tube having a memory portion comprised of a shape memory material and defining a normally unstressed curved configuration. The one delivery tube is longitudinally movable relative to the handle portion to extend the memory portion beyond the working channel of the endoscope such that the memory portion assumes the normal stressed curved configuration thereof. An electromagnetic probe is disposed within the delivery tube and is longitudinally movable relative to the delivery tube to extend a probe end portion thereof beyond the delivery tube and within tissue. The electromagnetic probe is adapted to follow the curved configuration of the memory portion of the delivery tube for deployment at an angularly oriented relation with respect to the endoscope. A rotatable control member is mounted to the handle portion and operatively connected to the delivery tube. The control member is rotatable to move the delivery tube between a first retracted position and a second advanced position. An actuator is also mounted to the handle portion and is operatively connected to the electromagnetic probe. The actuator is movable to extend the probe end portion beyond the delivery tube.

Preferred embodiments of the present invention will now be described by way of example only, with reference to the drawings, in which:
Figure 1 is a perspective view of a handle according to the present invention;
Figure 2 is a perspective view with parts separated of the handle of Figure 1;
Figure 3 is a side cross-sectional view of the handle in an unactuated position;
Figure 4 is a side plan view of the handle of Figure 1 mounted to a cytoscope;
Figure 5 is a view similar to the view of Figure 3 illustrating rotation of the control member to selectively deploy the delivery catheter;
Figure 6 is a view similar to the view of Figure 3 illustrating the deployment member advanced to deploy the electromagnetic probe;
Figure 7 is a cross-sectional view of the delivery catheter illustrating the outer sleeve, the probe delivery unit disposed within the outer sleeve and the electrodes disposed within the delivery tubes of the delivery unit;
Figure 8 is a side elevational view of the probe delivery unit;
Figure 9 is an axial view of the probe delivery unit as viewed from its proximal end;
Figure 10 is an axial view of the probe delivery unit as view from its distal end;
Figure 11 is a top elevational view of the probe deliver unit;
Figure 11A is a perspective view of the distal end of the probe delivery unit;
Figure 12 is a view illustrating a cytoscope on which the auxiliary thermal treatment apparatus of the invention may be mounted;
Figure 13 is a cross-sectional view taken along the lines 13-13 of Figure 12;
Figure 14 is an enlarged perspective view of the distal end portion of the cytoscope illustrating the delivery tubes of the probe delivery unit contained within the working channel of the scope;
Figure 15 is a view similar to the view of Figure 14 illustrating deployment of the distal end of the delivery tubes of the probe delivery unit whereby the distal end assumes its normal unstressed condition angularly oriented relative to the longitudinal axis of the apparatus;
Figure 16 is a side plan view of the distal end of the cytoscope in partial cross-sectioin further illustrating deployment of the delivery tubes with the electrodes in a retracted position disposed within the tubes;
Figures 17 is a view similar to the view of Figure 15 illustrating the electrodes in the advanced position;
Figure 18 is a view of an alternate embodiment of the auxiliary thermal treatment apparatus where a greater portion of the electrode is exposed to provide an increased thermal treatment capacity;
Figure 19 is a view of an alternate embodiment of the auxiliary thermal treatment apparatus incorporating a monopolar electrode assembly;
Figure 20 is a perspective view of the distal end of the electrode assembly with the monopolar electrode deployed beyond the distal end of the directional tube;
Figure 21 is a cross-sectional view of the electrode illustrating a thermocouple disposed within the electrode for detecting the temperature of the treatment area;
Figure 22 is a side plan view of the electrode and directional tube with the directional tube partially cut-away to illustrate a second thermocouple for detecting the temperature of the tissue adjacent the treatment area;
Figure 23 is a cross-sectional view taken along the lines 41-41 of Figure 22.

### DETAILED DESCRIPTION OF PREFERRED EMBODIMENTS

The apparatus of the present disclosure is intended to deliver electromagnetic energy to tissue for thermal treatment of the tissue including tissue ablation, tissue vaporization and/or tissue coagulation. The apparatus has particular application in the treatment of benign prostate hyperplasia (BPH) with electromagnetic radio frequency (RF) energy, however, it is to appreciated that the apparatus is not limited to such application. For example, the apparatus is not limited to the treatment of BPH, but, may be used in other surgical procedures such as cardiac ablation, cancer treatment, etc. Moreover, the apparatus may be used in any minimally invasive procedure where thermal treatment of tissue is desired and access to the tissue is limited.

The apparatus is particularly intended to be used in conjunction with an endoscope such as a cytoscope, fiber scope, laparoscope, urethroscope, etc. to provide the scope with thermal treatment capabilities. More specifically, the apparatus is at least partially insertable within the working channel of an endoscope, which is positioned in the body to access a targeted tissue area, to thermally treat the desired tissue.

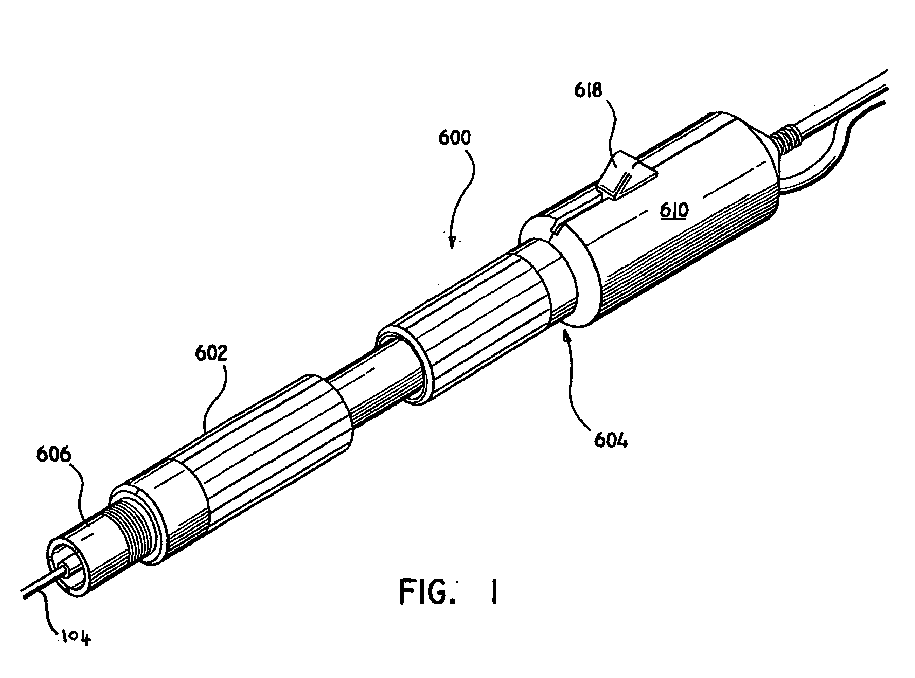

Referring now to Figures 1 to 3, there is illustrated a handle of the apparatus of the present disclosure. Elongated handle 600 includes stationary housing portion 602 and movable housing portion 604 which is longitudinal moveable relative to stationary housing portion 602. Stationary housing portion 602 has a mounting collar 606 mounted at its distal end which supports the elongate body of the apparatus. Stationary housing portion 602 further defines at its proximal end a threaded portion 608. Movable portion 604 includes a frame 610 and an elongated drive portion 612 extending from the frame 610. The elongated drive portion 612 is at least partially accommodated within the axial bore of stationary housing 602 and is adapted to move within the stationary housing 602 to deploy the delivery catheter as will be discussed.

A rotatable control member 614 is coaxially mounted about elongated drive portion 612. Rotatable control member 614 is longitudinally fixed with respect to movable housing portion 604 through an interfitting relationship of a locking groove and collar arrangement. More particularly, rotatable control member 614 includes a collar 615 which fits within a groove 617 of movable housing portion 604 to longitudinally fix the rotatable control member 614 to the movable housing portion 604. Rotatable control member 614 has an internal thread 616 which co-operates with threaded portion 608 of stationary housing 602 to longitudinally move the movable housing portion 604 upon rotation of the control member 614.

A deployment member 618 is mounted within the main frame 610 of movable housing 604 and is adapted to move longitudinally with respect to the movable housing portion 604. As will be appreciated from the description provided below, deployment member 618 is connected to the electromagnetic probe and functions in deploying the probe from the distal end of the delivery catheter.

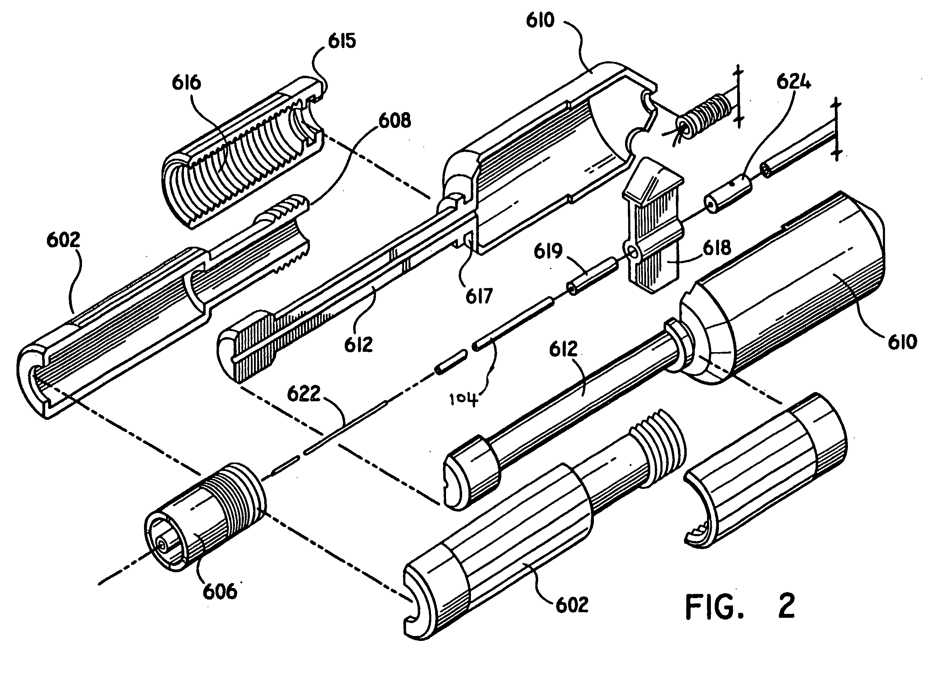

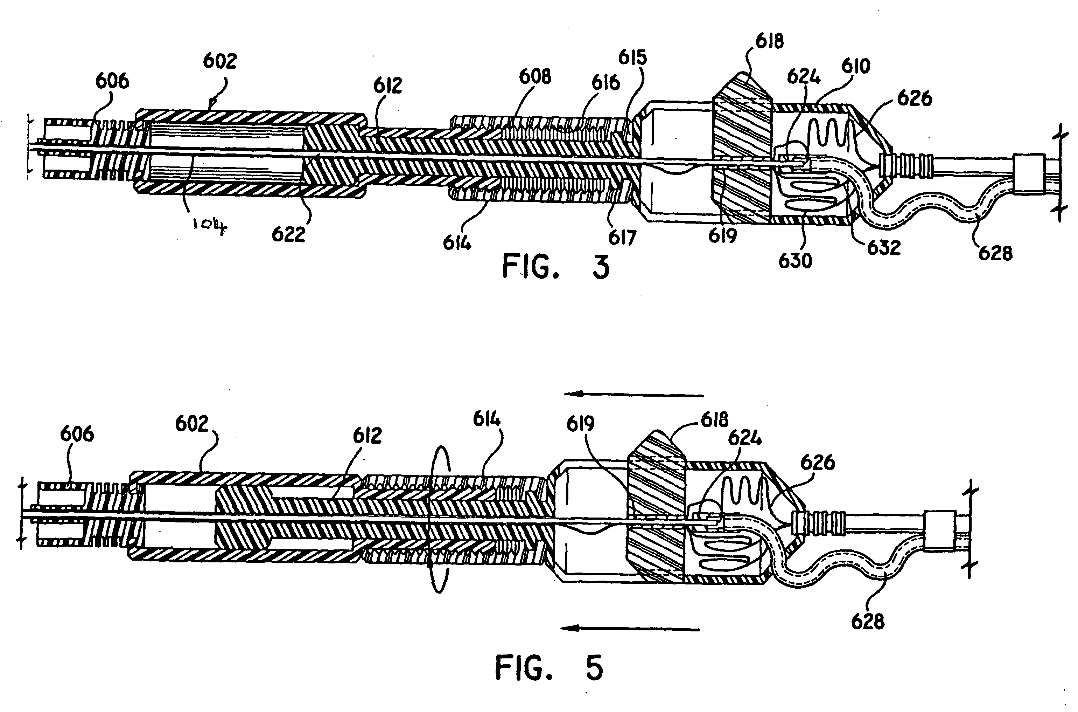

Referring particularly to Figure 3 in view of Figure 2, the interrelationship of the delivery catheter and electromagnetic probe with the components of handle 600 will be discussed. Delivery catheter 620 extends within the handle 600 and through an axial bore of movable housing 604. The proximal end of delivery catheter 620 is longitudinally fixed to elongated portion 612 of movable housing portion 604. Any conventional means for securing delivery catheter 620 to elongated drive portion 612 may be utilised including welding, cements, adhesives, etc. Accordingly, upon movement of movable housing portion 604 in the longitudinal direction as effectuated through rotation of rotatable control member 614, the delivery catheter 620 also moves longitudinally.

Electromagnetic probe 622 extends through delivery catheter 620 whereby the proximal end of the electromagnetic probe 622 continues within the main frame 610 of the movable housing portion 604. The proximal end of the electromagnetic probe 622 further extends through collar 619 mounted within deployment member 618 and terminates within a ferrule connector 624 disposed proximal of the deployment member 618. Electromagnetic probe 622 is longitudinally secured to collar 619 which is fixed to deployment member 618 such that movement of the deployment member causes corresponding longitudinal motion of the electromagnetic probe. Ferrule connector 624 may be any conventional connector and is preferably mounted within a longitudinal recess or groove defined in the frame of the movable housing portion 604. Ferrule connector 624 is fixed to the proximal end of electromagnetic probe 622 by conventional means including welding, cements, adhesives, etc. and serves to provide the electrical connection between the electromagnetic probe 622 and the service line or cable 626 which supplies the electromagnetic energy from the energy source. Ferrule connector 624 also serves in receiving the saline solution tube 628 to connect the tube to the interior lumen extending within the electromagnetic probe 622.

Also depicted in Figure 3 are the source lines servicing the two thermocouplers. In particular, the first line 630 services the thermocoupler extending between the outer shrink tubing and delivery catheter which detects or measures the temperature of tissue adjacent the tissue area. The second line 632 services the thermocoupler which extends within the electromagnetic probe 622 for detecting the temperature of the tissue in the treatment area.

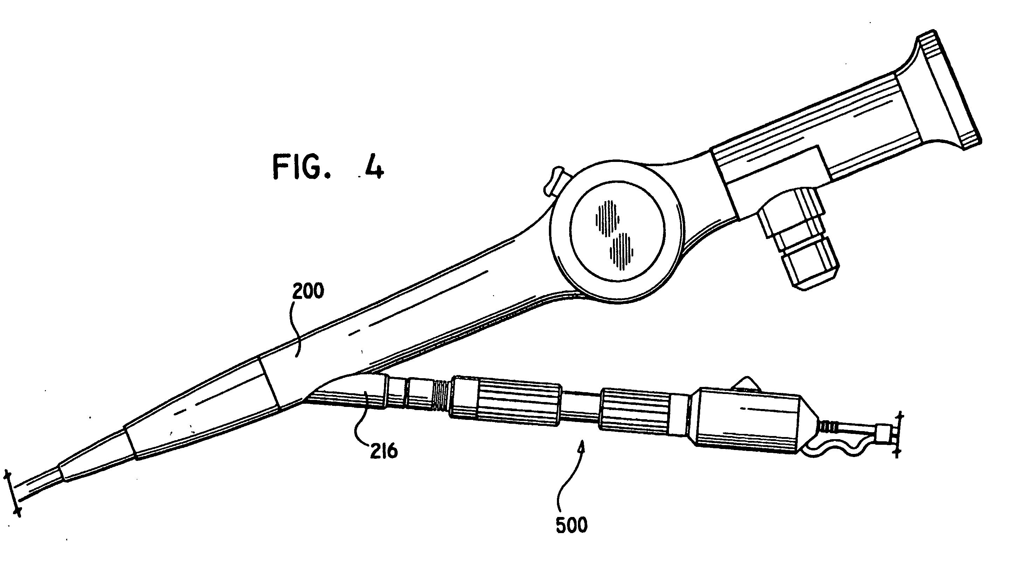

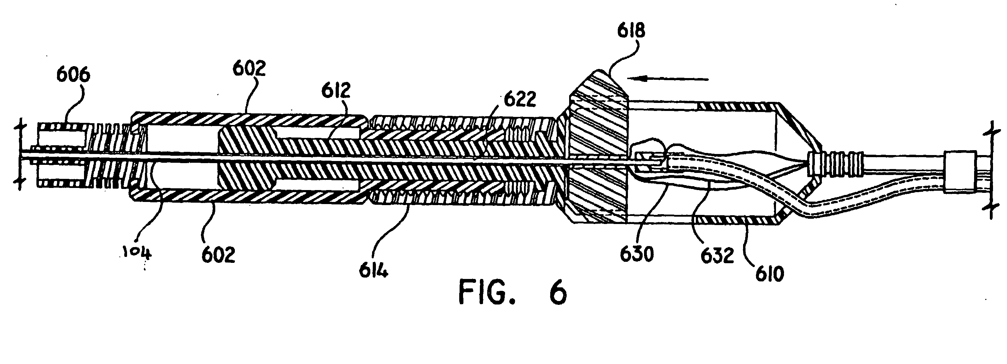

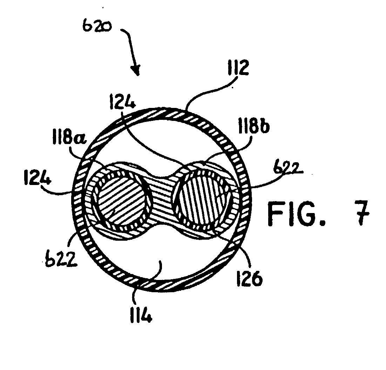

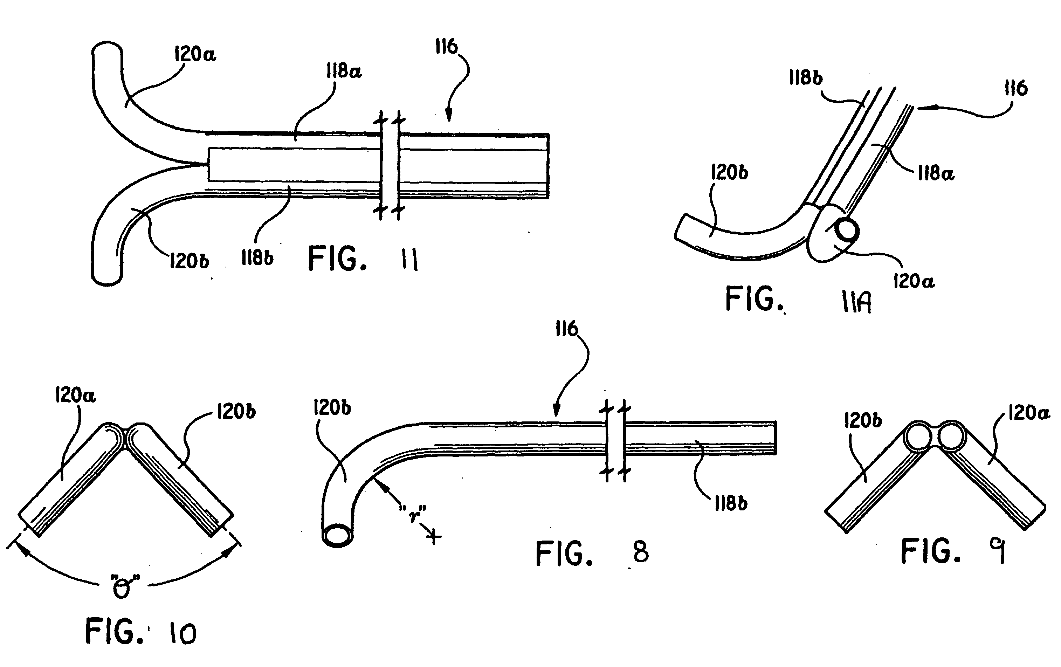

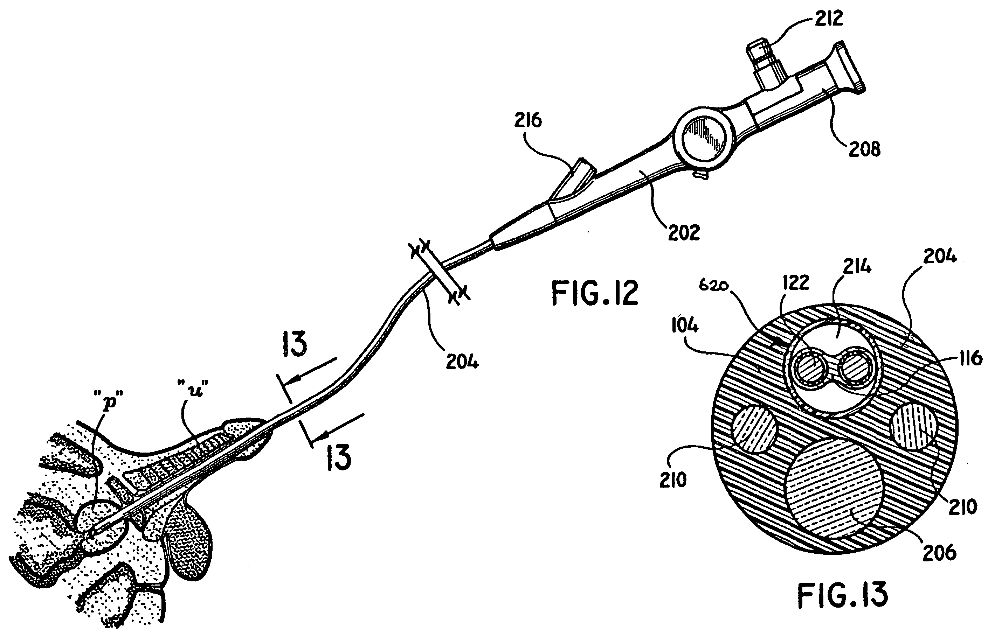

Referring now to Figure 4 the use of the apparatus will be discussed. With the cytoscope 200 accessing the urethral passage as discussed above, the elongated portion of the apparatus is inserted within the working channel of the scope and advanced until the handle engages the working channel port connector 216 extending from the proximal end of the working channel of the cytoscope as depicted in Figure 4. Preferably, handle 600 includes a Luer type connector at its end which releasably engages the port connector 216. With reference to Figure 5, the delivery catheter 620 is deployed by rotating the rotatable control member 614 in the direction depicted in Figure 5. As rotatable control member 614 rotates, the movable housing portion 604 advances through the threaded engagement of the threaded portions 608, 616 of rotatable control member 614 and the stationary housing 602 thereby advancing the delivery catheter 620 within the elongated portion of the apparatus and beyond the distal end of the working channel of the scope 200. It is appreciated that the rotatable control member 614 can be selectively incrementally rotated to provide selective incremental deployment of the delivery catheter 620, thus, providing enhanced control over the amount of deployment of the memory portion thereof. In effect, therefore, the angular orientation of the distal end of the delivery catheter 620 can be varied through the amount of deployment of the memory portion to achieve desired paths of entry into urethral tissue.

Once the delivery catheter 620 is deployed as desired, the electromagnetic probe 622 is deployed. With reference now to Figure 6, deployment member 618 is advanced in the direction of the directional arrow to deploy the electromagnetic probe 622 from the end of the delivery catheter. As the deployment member 618 moves in longitudinal direction, the ferrule connector 624 is also carried longitudinally due to the fixing of the proximal end of the electromagnetic probe and the ferrule connector 624 as discussed above. It is to be noted that the service lines 630, 632 servicing both the thermocouplers and the electromagnetic probe 622 have sufficient slack to permit advancing movement of the deployment member 618.

It is also envisioned that the auxiliary apparatus described above can be used other than with a scope. For example, the delivery (directing) tubes can be inserted directly into the urethra or other body lumens. The tubes and electrodes can be monitored by ultrasound, MRI, fluoroscopy or other imaging techniques. Ultrasound can also be used in conjunction with the endoscope to image the needles in the edenoma.

As shown in Figure 7, delivery catheter 620 may include outer sleeve 112 preferably fabricated from a flexible material such as Nitinol. It is envisioned that outer sleeve 112 may alternately be rigid if, for example, it is intended to be used with a rigid scope. Outer sleeve 112, if provided, ranges from about 25 to about 40 millimeters (mm) in length, preferably, about 37mm and ranges from about 1.5 to about 2.5 millimeters in diameter, preferably about 2.3 mm. Outer sleeve 112 defines axial bore 114 extending therethrough. Other dimensions are also contemplated. Alternatively, the outer sleeve may be eliminated.

Referring now to Figures 7 to 11A, probe delivery unit, identified generally by reference numeral 116, is disposed within axial opening 114 of outer sleeve 112. Probe guide 116 is adapted for reciprocal longitudinal movement within the opening 114 and includes first and second hollow delivery (directional) tubes 118a, 118b. Delivery tubes 118a, 118b are preferably connected to each other for a major portion of their respective lengths, but are separated at the distal end portions 120a, 120b as best depicted in Figures 11 and 11a. Delivery tubes 118a, 118b accommodate electromagnetic probes 122 therein and function in guiding the probes 622 at desired orientations within the tissue.

Referring particularly to Figures 8 to 11A, delivery tubes (or catheter) 118a, 118b of probe guide 116 are preferably fabricated from a shape memory metal such as NITINOL and are preferably joined at each other by welding or with the use of adhesives. In the normal condition of delivery tubes 118a, 118b, the distal ends 120a, 120b of the tubes 118a, 118b each assume the arcuate configuration depicted in Figures 8 to 11A, i.e. the distal end portions 120a, 120b have memory to define the arcuate orientation as shown, thus, providing arcuate paths for electromagnetic probes 622 to follow to penetrate the tissue. The particular orientation of memory portions 120, 120b of delivery tubes 118a, 118b can be varied depending on the objectives of the surgical procedure. The distal end or memory portions 120a, 120b of delivery tubes 118a, 118b readily adopt a linear configuration when confined in the outer sleeve 112 of elongated portion 104 as will be discussed.

In a preferred embodiment (e.g. in BPH application), memory portions 120a, 120b of delivery tubes 118a, 118b define a radius of curvature "r" ranging between about (.250) 6.35mm to about (.400 inches) 10.16mm, preferably about (.312 inches) 7.93mm. Memory portions 120a, 120b are also separated by an angle "T" ranging from about 45° to about 90° (degrees). Clearly other dimensions and angular orientations of memory portions 120a, 120b are contemplated as well.

With reference again to Figure 7, electromagnetic probe 622 disposed within delivery tubes 118a, 118b include bipolar electrodes formed of a thin solid wire capable of carrying an electromagnetic radiofreqeuncy (RF) current. The electrodes are relatively flexible to follow along the path defined by delivery tubes 118a, 118b, but sufficient in rigidity to be advanced into tissue. The electrodes are preferably made of Nitinol so they can return to their normal straight configuration after being bent by the delivery tubes. The electrodes each have a pointed tip to facilitate penetration through the tissue. Each electrode has an insulating layer, designated by reference numeral 124, which extends along a major portion of its length to prevent damage to non-targeted body tissue. Each electrode is therefore electrically isolated from its delivery tube. Insulating layer 124 terminates to expose the distal penetrating portions of the electrodes 622, thus, permitting the transmission of electromagnetic RF current to the targeted body tissue. Alternatively, monopolar electrodes could be provided.

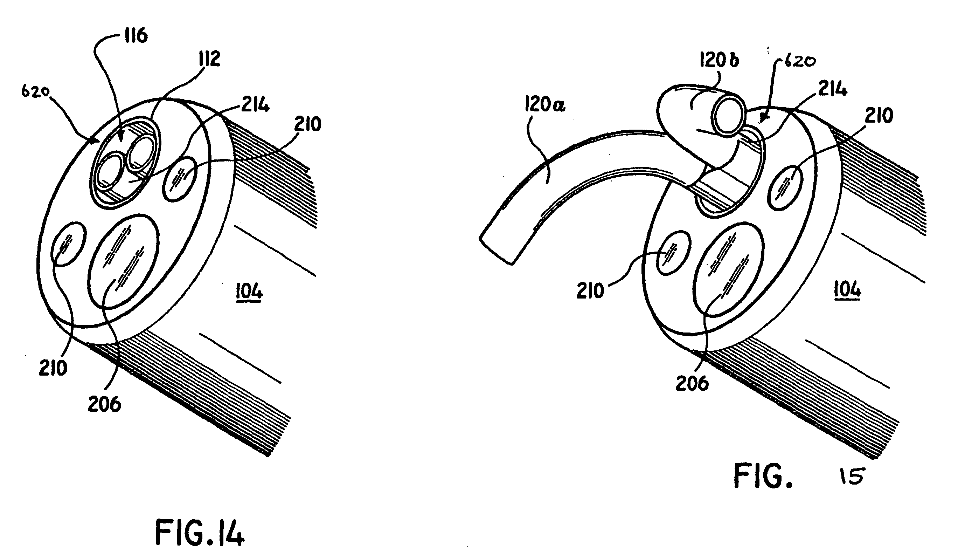

Apparatus 600 can be positioned within a conventional cytoscope for thermal treatment of prostate "p" to alleviate the symptoms of BPH. One conventional cytoscope with which the apparatus of the present disclosure can be utilised is the CAN Flexible CystoNephroscope manufactured by Circon ACMI. As shown in Figure 12, cytoscope 200 includes handle 202 and a flexible elongated portion 204 connected to the handle 202 and extending distally therefrom. Cytoscope 200 incorporates an optical system to permit viewing of the tissue to be treated. As depicted in Figure 13, the optical system preferably consists of flexible fiber optic bundles (identified by reference numeral 206) which are accommodated within a longitudinal bore extending through the elongated portion 204 of the scope 200. The fiber optic bundles 206 extend to eyepiece 208 where the surgeon can view the image transmitted by the optical system.

Cytoscope 200 also includes an illumination system which provides illuminating light to the targeted tissue area. The illumination system includes a plurality of optical fibers 210 which are accommodated within a plurality of longitudinal channels (two are shown) of elongated portion 204 and extend within handle 202 where they terminate at illumination coupler 212. Illumination coupler 212 is connectable to a conventional light source as is known in the art. Cytoscope 200 further includes a working channel 214 extending through flexible elongated portion 204 and terminating at channel port 216 of handle 202. Working channel 214 is adapted to receive various surgical instrumentation through channel port 216 (e.g. thermal treatment apparatus 600) to permit the performance of surgical procedures at the distal end of the cystoscope 200. Cystoscope 200 is preferably a 5mm scope.

### Operation

The use of apparatus 600 with cystoscope 200 in conjunction with the thermal treatment of prostatic tissue will now be discussed. Cystoscope 200 is inserted through urethral passage "u" of the patient and advanced within the passage until the distal end of the scope is adjacent prostate gland "p". Thereafter, delivery catheter 620 of apparatus 600 is inserted into working channel 214 of cystoscope 200 and advanced into the working channel 214 until apparatus 600 contacts channel port 216 of scope handle 202. As an alternative method of insertion, apparatus 600 may be positioned within cystoscope 200 prior to insertion within the urethral passage "u" and the entire assembly may be then advanced within the urethral passage. It is envisioned that apparatus 600 may incorporate a locking mechanism to lockingly engage channel port 216 of handle 202 of the cystoscope 200.

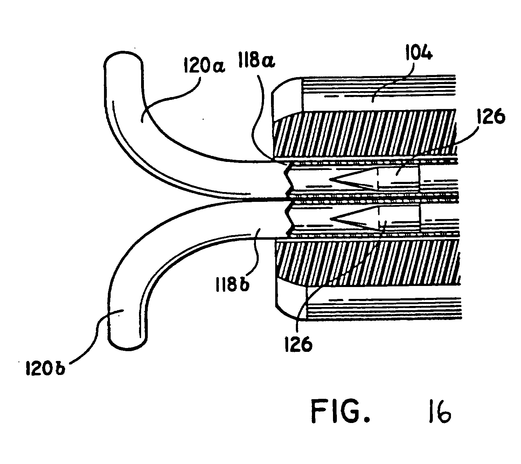

With reference now to Figure 14, probe delivery unit 116 is shown in its retracted position. In such position, the distal end portions 120a, 120b of delivery tubes 118a, 118b are constrained by outer sleeve 112 (and elongated portion 204 of scope 200) thereby assuming a general linear configuration within the sleeve 112. Thereafter, the apparatus 600 is operated to move probe delivery unit from its retracted position of Figure 14 to its extended position of Figure 15. Upon exiting working channel 214 of cystoscope 200, the distal end or memory portions 120a, 120b of delivery tubes 118a, 118b are no longer constrained by outer sleeve 112, and, thus are free to assume their normal unstressed curved configurations depicted in Figure 15. By exiting through the distal end face of the working channel 214 of cystoscope 200, the deployment of delivery tubes 118a, 118b can be monitored with the optical system of cystocscope 200. That is, both 0 degree and oblique viewing is achieved. In the extended position of delivery tubes 118a, 118b, the distal end portions 120a, 120b may slightly extend beyond the outer circumference of scope 200, but, however, do not penetrate the urethral lining. It is to be noted that the degree of deployment of memory portions 120a, 120b of delivery tubes 118a, 118b may be selected to thereby achieve desired angular orientations of the memory portions 120a, 120b, consequently, controlling the orientation of the deployed electrodes. (As noted above, alternately, outer sleeve 112 need not be provided and the apparatus is advanced through the working channel to expose the delivery tubes.)

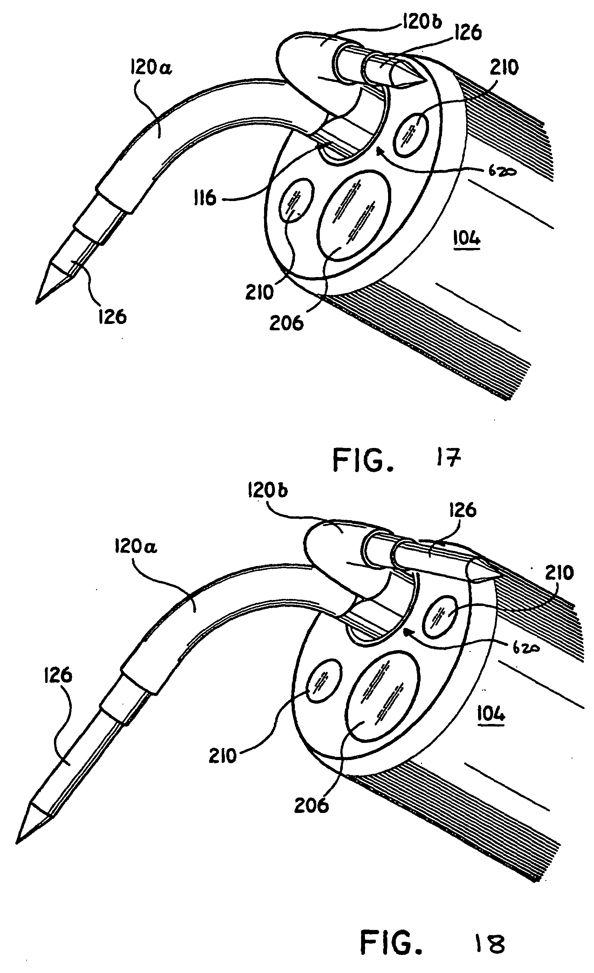

Referring now to Figures 16 and 17, with distal end portions 120a, 120b in their extended positions, attention is directed to deploying the electromagnetic probes 622. Figure 16 depicts the electromagnetic probes 622 in their retracted position within delivery tubes 118a, 118b. Second actuator 110 is selectively distally advanced to advance electromagnetic probe 622 from delivery tubes 118a, 118b. During advancing movement, the penetrating end portions 126 of probes 622 flex or bend to conform to the curved configuration of memory portions 122a, 122b of the delivery tubes 118a, 118b to pierce the urethral wall "u" and enter the prosthetic tissue "p". The degree of deployment of electromagnetic probes 622 may be selectively controlled (e.g. partial deployment) with second actuator 110 to thereby provide a level of control over the thermal treatment field generated by the probe.

The system is then energised to thermally treat (e.g. ablate, vaporise or cauterise) the desired prosthetic tissue with RF energy. As a result of this treatment, the prosthetic tissue BPH necroses and dies, thus, relieving pressure off the urethral wall and alleviating the symptoms of BPH. During treatment, the depth of penetration of penetrating end portions 126 of electromagnetic probes 622 may be selectively adjusted using apparatus 600 to permit specific regions of the prosthetic tissue "p" to be targeted for thermal treatment thus providing heating pattern flexibility and control. During treatment, insulating layer 124 of electromagnetic probes 622 preferably contact the urethral wall "u" to prevent damage to the wall.

Upon completion of the procedure, the system is de-energised and the cystoscope 200 and apparatus are removed from the urethral passage "u".

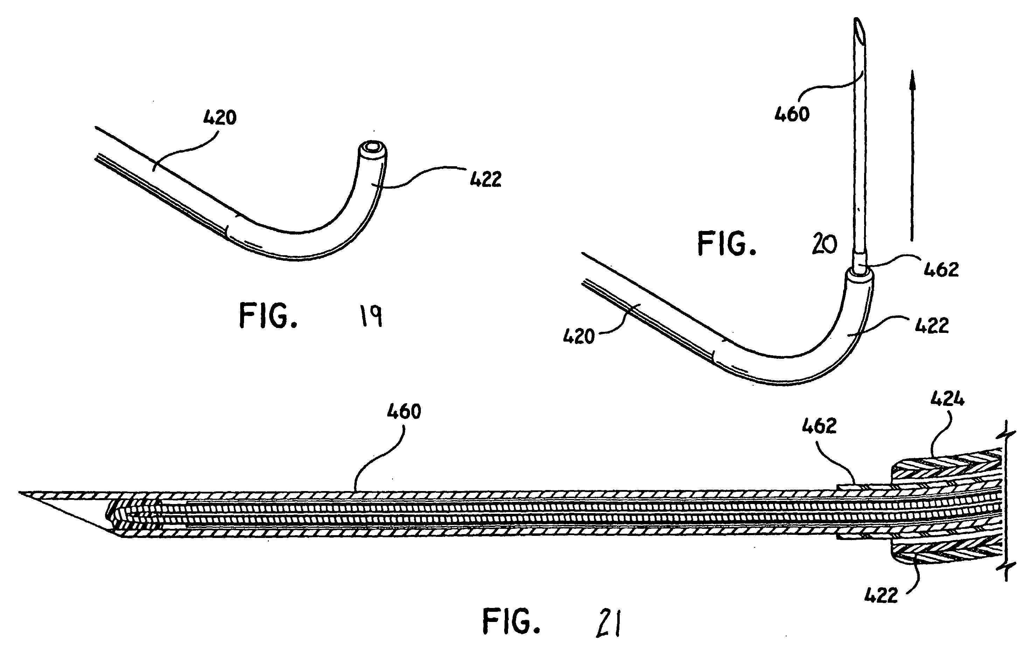

Figure 18 is a perspective view of the distal end of cystoscope 200 with an alternate auxiliary thermal treatment apparatus mounted within the working channel 214 of the scope. It is to be appreciated that the lengths of the exposed electrode portions, i.e. the length of insulation, may be varied to achieve desired thermal treatment objectives.

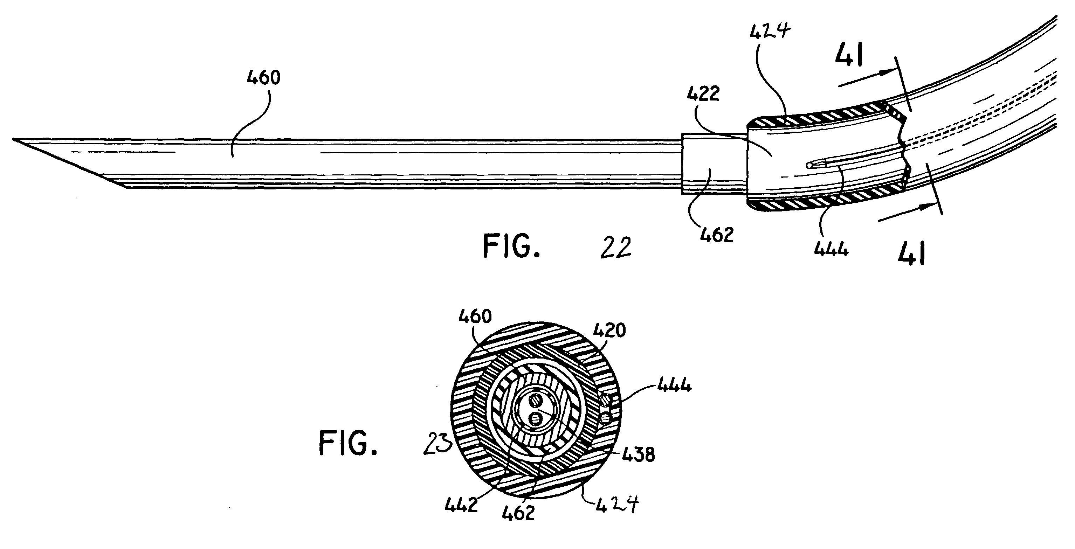

Figures 19 to 23 illustrate an alternate embodiment of the auxiliary thermal treatment apparatus of Figure 18. This apparatus is similar in most respects to the apparatus of Figure 18, but, incorporates a monopolar electrode assembly having a single monopolar electrode 460 with insulating layer 462. The apparatus may be utilised with a grounding pad positioned adjacent the body as is conventional in the art. Delivery catheter 420 and memory portion 422 are substantially similar to the prior embodiment. A shrink tubing 424 is positioned about delivery catheter 420. As best depicted in Figures 22 to 23, thermocouple 438 is disposed within delivery catheter 420 and thermocouple 444 is disposed between the shrink tubing 424 and the outer surface of delivery catheter 420.

## Claims

1. An apparatus for the thermal treatment of tissue comprising a housing (600) dimensioned to be grasped with the hand of a user, and an elongate portion (104) connected to the housing (600) extending distally therefrom, and dimensioned for insertion within a narrow body passage and defining a longitudinal axis, said elongate portion (104) including at least one delivery catheter (620, 420) having a distal end, the or each delivery catheter (620, 420) being movable relative to the housing (600) in a generally longitudinal direction between a retracted position and an advanced position, an electromagnetic probe (622) disposed within the or each delivery catheter (620, 420) and movable in a general longitudinal direction therein to extend an end portion (126)thereof beyond the distal end of the delivery catheter (620, 420) and within the tissue, the electromagnetic probe (622) being adapted to follow a path defined by the respective delivery catheter (620, 420) and being connected to a thermal energy source, and a rotatable control member (614) mounted to the housing (602, 604) and operatively connected to the delivery catheter (620, 420) operable to move the delivery catheter (620, 420) between the retracted position and the advanced position, **characterised in that** the housing (600) is divided into a stationary portion (602) having an externally threaded portion (608) and a movable portion (604) including an annular groove (617) and a deployment member (618) for the electromagnetic probe (622), the control member (614) having an internal thread (616) for engagement with the externally threaded portion (608) and being longitudinally fixed to the movable portion (604) of the housing (600) by means of a collar (615) in engagement with the groove (617), rotation of the control member (614) causing movement of the delivery catheter (620, 420) between the retracted and the advanced position.

2. The apparatus of claim 1 **characterised in that** the electromagnetic probe (622) is a monopolar electrode (460).

3. The apparatus according to claim 1 or claim 2 **characterised in that** the delivery catheter (620, 420) includes a memory portion (422) disposed ad the distal end thereof, the memory portion (422) comprised of shape memory material and defining an arcuate configuration when in a normal unstressed condition thereof.

4. The apparatus according to any of the preceding claims wherein the deployment member (618) is operatively connected to the electromagnetic probe (622) and is movable to deploy the probe end portion of the electromagnetic probe (622) from the delivery catheter (620, 420).

5. The apparatus according to any of the preceding claims wherein the delivery catheter (620, 420) includes an outer sleeve (112) which accommodates the electromagnetic probe (622).

6. The apparatus according to any of the preceding claims **characterised in that** the control member (614) is operatively connected to the electromagnetic probe (622) such that movement of the control member (614) to move the delivery catheter (620, 420) between the retracted and advanced positions causes corresponding longitudinal movement of the electromagnetic probe (622).

7. The apparatus according to any of the preceding claims **characterised by** a thermocouple (438) for detecting temperature of tissue within a treatment area generated by the electromagnetic probe (622).

8. The apparatus according to any of the preceding claims **characterised by** a thermocouple (444) for detecting temperature of tissue adjacent a treatment area generated by the electromagnetic probe (622).

9. The apparatus according to any of the preceding claim **characterised in that** the elongate portion (104) is dimensioned for insertion within a working channel of a cystoscope (200).

10. The apparatus according to any of the preceding claims wherein the delivery catheter (620, 420) includes a flexible outer sleeve (112), the one delivery catheter being at least partially disposed within the outer sleeve (112).

11. A system for the thermal treatment of tissue including an apparatus for the thermal treatment of tissue according to claim 1 and further comprising an endoscope including an elongate body (204) having a working channel, the elongate portion (104) of the apparatus being dimensioned to be positioned within the working channel of the endoscope, the electromagnetic probe (622) of the apparatus being adapted to follow the angularly offset configuration of the distal end of the delivery catheter (620, 420) when in the advanced position thereof, the control member (614) being operable to move the delivery catheter (620, 420) within the working channel of the endoscope from the retracted to the advanced position to thereby deploy the distal end of the delivery catheter (620, 420) from the working channel such that a memory portion (122a, 122b) disposed at the distal end portion thereof which is made of shape memory material defining an arcuate configuration when in normal unstressed condition assumes its normal unstressed curved configuration.

12. A system according to claim 11 **characterised in that** the working channel of the endoscopic portion of the endoscope includes an axial bore extending through the distal end face of the end endoscopic portion and wherein the elongate portion (104) of the thermal treatment device includes an axial bore extending through the distal end face of the elongate body (204), the delivery catheter (620, 420) and electromagnetic probe (622) being through the distal end face of the elongate portion (104).

13. A system according to claim 11 or claim 12 **characterised in that** the elongate portion (104) is operatively connected to a source of dissipating agent for facilitating dissipation of thermal energy at the treatment site and wherein the dissipating agent is conveyed through a channel extending through the elongate portion (104).

14. The system according to claim 13 **characterised in that** the electromagnetic probe (622) defines a hollow passageway, the passageway defining a channel for passage of the dissipating agent.

15. The system according to any of claims 11 to 14 **characterised in that** the endoscope includes an optical system (206) for viewing an image of an object and an illumination system (210) for providing illuminating light.

## Patentansprüche

1. Vorrichtung zur thermischen Behandlung von Gewebe, umfassend ein Gehäuse (600), das so dimensioniert ist, dass es von einem Benutzer in die Hand genommen werden kann, und einen länglichen Abschnitt (104), der mit dem Gehäuse (600) verbunden ist und sich distal von diesem erstreckt und zum Einführen in einen engen Körperkanal dimensioniert ist und eine Längsachse definiert, wobei der längliche Abschnitt (104) wenigstens einen Zuführungskatheter (620, 420) mit einem distalen Ende enthält, wobei der oder jeder Zuführungskatheter (620, 420) in bezug auf das Gehäuse (600) im wesentlichen in einer Längsrichtung zwischen einer zurückgezogenen Position und einer vorgeschobenen Position bewegbar ist, eine elektromagnetische Sonde (622) in dem oder jedem Zuführungskatheter (620, 420) angeordnet und im wesentlichen in Längsrichtung in diesem bewegbar ist, um einen Endabschnitt (126) desselben über das distale Ende des Zuführungskatheters (620, 420) hinaus und in dem Gewebe auszufahren, und die elektromagnetische Sonde (622) so ausgebildet ist, dass sie einem Pfad folgt, der durch den entsprechenden Zuführungskatheter (620, 420) definiert ist und mit einer Wärmeenergiequelle verbunden ist, und ein drehbares Steuerelement (614) an dem Gehäuse (602, 604) angebracht und mit dem Zuführungskatheter (620, 420) wirkverbunden ist, das betätigbar ist, um den Zuführungskatheter (620, 420) zwischen der zurückgezogenen Position und der vorgeschobenen Position zu bewegen, **dadurch gekennzeichnet, dass** das Gehäuse (600) in einen feststehenden Abschnitt (602) mit einem Außengewindeabschnitt (608) und einen beweglichen Abschnitt (604) mit einer ringförmigen Rille (617) und einem Betätigungselement (618) für die elektromagnetische Sonde (622) unterteilt ist, wobei das Steuerelement (614) ein Innengewinde (616) zum Eingriff mit dem Außengewindeabschnitt (608) aufweist und in Längsrichtung an dem beweglichen Teil (604) des Gehäuses (600) durch einen Kragen (615) festgelegt ist, der mit der Rille (617) in Eingriff ist, wobei die Drehung des Steuerelements (614) eine Bewegung des Zuführungskatheters (620, 420) zwischen der zurückgezogenen und der vorgeschobenen Position bewirkt.

2. Vorrichtung nach Anspruch 1, **dadurch gekennzeichnet, dass** die elektromagnetische Sonde (622) eine monopolare Elektrode (460) ist.

3. Vorrichtung nach Anspruch 1 oder 2, **dadurch gekennzeichnet, dass** der Zuführungskatheter (620, 420) einen Formgedächtnisabschnitt (422) enthält, der an seinem distalen Ende angeordnet ist, wobei der Formgedächtnisabschnitt (422) aus einem Formgedächtnismaterial besteht und eine gekrümmte Form definiert, wenn es sich in einem normalen, ungespannten Zustand befindet.

4. Vorrichtung nach einem der vorangehenden Ansprüche, wobei das Betätigungselement (618) mit der elektromagnetischen Sonde (622) wirkverbunden und bewegbar ist, um den Sondenendabschnitt der elektromagnetischen Sonde (622) von dem Zuführungskatheter (620, 420) aus einzusetzen.

5. Vorrichtung nach einem der vorangehenden Ansprüche, wobei der Zuführungskatheter (620, 420) eine äußere Hülse (112) enthält, welche die elektromagnetische Sonde (622) aufnimmt.

6. Vorrichtung nach einem der vorangehenden Ansprüche, **dadurch gekennzeichnet, dass** das Steuerelement (614) mit der elektromagnetischen Sonde (622) wirkverbunden ist, so dass die Bewegung des Steuerelements (614) zur Bewegung des Zuführungskatheters (620, 420) zwischen der zurückgezogenen und der vorgeschobenen Position eine entsprechende Längsbewegung der elektromagnetischen Sonde (622) bewirkt.

7. Vorrichtung nach einem der vorangehenden Ansprüche, **gekennzeichnet durch** ein Thermoelement (438) zum Erfassen der Gewebetemperatur in einem Behandlungsareal, welches von der elektromagnetischen Sonde (622) erzeugt wird.

8. Vorrichtung nach einem der vorangehenden Ansprüche, **gekennzeichnet durch** ein Thermoelement (444) zum Erfassen der Gewebetemperatur neben einem Behandlungsareal, welches von der elektromagnetischen Sonde (622) erzeugt wird.

9. Vorrichtung nach einem der vorangehenden Ansprüche, **dadurch gekennzeichnet, dass** der längliche Abschnitt (104) zum Einsetzen in einen Arbeitskanal eines Zystoskops (200) dimensioniert ist.

10. Vorrichtung nach einem der vorangehenden Ansprüche, wobei der Zuführungskatheter (620, 420) eine flexible äußere Hülse (112) enthält, wobei der eine_Zuführungskatheter zumindest teilweise in der äußeren Hülse (112) angeordnet ist.

11. System zur thermischen Behandlung von Gewebe, enthaltend eine Vorrichtung zur thermischen Behandlung von Gewebe nach Anspruch 1 und ferner umfassend ein Endoskop, das einen länglichen Körper (204) mit einem Arbeitskanal enthält, wobei der längliche Abschnitt (104) der Vorrichtung so dimensioniert ist, dass er in dem Arbeitskanal des Endoskops positioniert wird, die elektromagnetische Sonde (622) der Vorrichtung dazu ausgebildet ist, der winkeligen Versatzform des distalen Endes des Zuführungskatheters (620, 420) zu folgen, wenn sie in der vorgeschobenen Position ist, das Steuerelement (614) betätigbar ist, um den Zuführungskatheter (620, 420) in dem Arbeitskanal des Endoskops aus der zurückgezogenen in die vorgeschobenen Position zu bewegen, um dadurch das distale Ende des Zuführungskatheters (620, 420) von dem Arbeitskanal auszusetzen, so dass ein Formgedächtnisabschnitt (122a, 122b), der an dem distalen Endabschnitt angeordnet ist, der aus Formgedächtnismaterial besteht, das eine bogenförmige Form im normalen ungespannten Zustand definiert, seine normale ungespannte gekrümmte Form einnimmt.

12. System nach Anspruch 11, **dadurch gekennzeichnet, dass** der Arbeitskanal des endoskopischen Abschnitts des Endoskops eine Axialbohrung enthält, die sich durch die distale Stirnfläche des endoskopischen Endabschnitts erstreckt, und wobei der längliche Abschnitt (104) der thermischen Behandlungsvorrichtung eine Axialbohrung enthält, die sich durch die distale Stirnfläche des länglichen Körpers (204) erstreckt, wobei der Zuführungskatheter (620, 420) und die elektromagnetische Sonde (622) durch die distale Stirnfläche des länglichen Abschnitts (104) gehen.

13. System nach Anspruch 11 oder Anspruch 12, **dadurch gekennzeichnet, dass** der längliche Abschnitt (104) betriebsmäßig mit einer Quelle für ein Dissipationsmittel verbunden sind, um die Ableitung von Wärmeenergie an der Behandlungsstelle zu erleichtern, und wobei das Dissipationsmittel durch einen Kanal geleitet wird, der sich durch den länglichen Abschnitt (104) erstreckt.

14. System nach Anspruch 13, **dadurch gekennzeichnet, dass** die elektromagnetische Sonde (622) einen hohlen Durchlass definiert, wobei der Durchlass einen Kanal für den Durchgang des Dissipationsmittels definiert.

15. System nach einem der Ansprüche 11 bis 14, **dadurch gekennzeichnet, dass** das Endoskop ein optisches System (206) zur Bildbetrachtung eines Objekts und ein Beleuchtungssystem (210) zur Bereitstellung von Beleuchtungslicht enthält.

## Revendications

1. Un appareil pour le traitement thermique d'un tissu, comprenant un boîtier (600) dimensionné de manière à pouvoir être pris à la main par un utilisateur et une portion allongée (104) réunie au boîtier (600) et s'en étendant distalement et dimensionnée de manière à pouvoir être insérée dans un passage étroit du corps, et définissant un axe longitudinal, ladite portion allongée (104) comportant au moins un cathéter de délivrance (620, 420) ayant une extrémité distale, le ou chaque cathéter de délivrance (620, 420) pouvant être déplacé par rapport au boîtier (600) dans une direction générale longitudinale entre une position rétractée et une position avancée, une sonde électromagnétique (622) disposée à l'intérieur du ou de chaque cathéter de délivrance (620, 420) et pouvant être déplacée dans une direction générale longitudinale à l'intérieur de celui-ci de manière à s'étendre depuis une position finale (126) de celui-ci au-delà de l'extrémité distale du cathéter de délivrance (620, 420) et à l'intérieur du tissu, la sonde électromagnétique (622) étant agencée de manière à suivre un trajet défini par le cathéter de délivrance correspondant (620, 420) et étant connectée à une source d'énergie thermique et un organe de contrôle rotatif (614) monté sur le boîtier (602, 604), réuni positivement au cathéter de délivrance (620, 420) et pouvant être actionné de manière à déplacer le cathéter de délivrance (620, 420) entre la position rétractée et la position avancée, **caractérisé en ce que** le boîtier (600) est divisé en une portion fixe (602) présentant une portion filetée extérieurement (608) et une portion mobile (604) présentant une rainure annulaire (617) et un organe de déploiement (618) pour la sonde électromagnétique (622), l'organe de contrôle (614) présentant un filetage inteme (616) destiné à coopérer avec la portion à filetage externe (608) et étant fixée longitudinalement à la portion déplaçable (604) du boîtier (600) au moyen d'un collier (615) coopérant avec la rainure (617), la rotation de l'organe de contrôle (614) provoquant le déplacement du cathéter de délivrance (620, 420) entre la position rétractée et la position avancée.

2. L'appareil de la revendication 1, **caractérisé en ce que** la sonde électromagnétique (622) est une électrode monopolaire (460).

3. L'appareil selon la revendication 1 ou la revendication 2, **caractérisé en ce que** le cathéter de délivrance (620, 420) comporte une portion à mémoire (422) disposée à son extrémité distale, la portion à mémoire (422) étant réalisée en un matériau à mémoire de forme et définissant une configuration arrondie quand elle est en condition non contrainte normale.

4. L'appareil selon l'une quelconque des revendications précédentes, dans lequel l'organe de déploiement (618) est connecté positivement à la sonde électromagnétique (622) et peut être déplacé de manière à déployer l'extrémité constituant la sonde électromagnétique (622) du cathéter depuis le cathéter de délivrance (620, 420).

5. L'appareil selon l'une quelconque des revendications précédentes, dans lequel le cathéter de délivrance (620, 420) comporte un manchon extérieur (112) qui reçoit la sonde électromagnétique (622).

6. L'appareil selon l'une quelconque des revendications précédentes, **caractérisé en ce que** l'organe de contrôle (614) est connecté positivement à la sonde électromagnétique (622) d'une manière telle que le déplacement de l'organe de contrôle (614) pour déplacer le cathéter de délivrance (620, 420) entre les positions rétractées et avancées provoque le déplacement longitudinal correspondant de la sonde électromagnétique (622).

7. L'appareil selon l'une quelconque des revendications précédentes, **caractérisé par** un thermocouple (438) destiné à détecter la température du tissu à l'intérieur d'une zone de traitement générée par la sonde électromagnétique (622).

8. L'appareil selon l'une quelconque des revendications précédentes, **caractérisé par** un thermocouple (444) destiné à détecter la température d'un tissu adjacent à une zone de traitement générée par la soride électromagnétique (622).

9. L'appareil selon l'une quelconque des revendications précédentes, **caractérisé en ce que** la portion allongée (104) est dimensionnée de manière à pouvoir être insérée à l'intérieur d'un canal de travail d'un cystoscope (200).

10. L'appareil selon l'une quelconque des revendications précédentes, dans lequel le cathéter de délivrance (620, 420) comporte un manchon extérieur souple (112), ce cathéter de délivrance étant au moins en partie disposé à l'intérieur du manchon extérieur (112).

11. Un système pour le traitement thermique de tissu, comprenant un appareil pour le traitement thermique de tissu selon la revendication 1, et comprenant au surplus un endoscope se composant d'un corps allongé (204) présentant un canal de travail, la portion allongée (104) de l'appareil étant dimensionnée de manière à être insérée à l'intérieur du canal de travail de l'endoscope, la sonde électromagnétique (622) de l'appareil étant agencée de manière à suivre la configuration angulairement décalée de l'extrémité distale du cathéter de délivrance (620, 420) dans la position avancée de celui-ci, l'organe de contrôle (614) pouvant être actionné de manière à déplacer le cathéter de délivrance (620, 420) à l'intérieur du canal de travail de l'endoscope depuis la position rétractée jusqu'à la position avancée, de manière à déployer ainsi l'extrémité distale du cathéter de délivrance (620, 420) depuis le canal de travail d'une manière telle qu'une portion à mémoire (122a, 122b) disposée à l'extrémité distale de celui-ci, qui est réalisé en un matériau à mémoire de forme définissant une configuration arrondie quand, en condition normale non contrainte, il assume sa configuration arrondie normale non contrainte.

12. Un système selon la revendication 11, **caractérisé en ce que** le canal de travail de la portion endoscopique de l'endoscope comprend un alésage axial s'étendant à travers l'extrémité distale de la portion endoscopique terminale et dans lequel la portion allongée (104) du dispositif de traitement thermique comporte un alésage axial s'étendant à travers l'extrémité distale du corps allongé (204), le cathéter de délivrance (620, 420) et la sonde électromagnétique (622) étant situés à travers la face terminale distale de la portion allongée (104).

13. Un système selon la revendication 11 ou la revendication 12, **caractérisé en ce que** la portion allongée (104) est connectée positivement à une source d'un agent dissipant facilitant la dissipation de l'énergie thermique au site de traitement et dans lequel l'agent dissipant est amené par un canal s'étendant à travers la portion allongée (104).

14. Le système selon la revendication 13, **caractérisé en ce que** la sonde électromagnétique définit un passage creux, ce passage constituant un canal pour le passage de l'agent dissipant.

15. Le système selon l'une quelconque des revendications 11 à 14, **caractérisé en ce que** l'endoscope comporte un système optique (206) pour l'observation d'une image d'un objet et un système d'illumination (210) assurant une lumière d'illumination.
